# EUROPEAN PATENT SPECIFICATION

(11) **EP 0 889 040 B1**
(45) Date of publication and mention of the grant of the patent: **16.04.2003**
(21) Application number: 98109168.9
(22) Date of filing: 20.05.1998
(51) Int. Cl.: C07D 263/44

(54) **Process for producing 2,4-oxazolidinedione**
Verfahren zur Herstellung von 2,4-Oxazolidindion
Procédé pour la préparation d'oxazolidinedione-(2,4)

(30) Priority: 30.06.1997 JP 17477297
(43) Date of publication of application: 07.01.1999
(73) Proprietor: MITSUBISHI GAS CHEMICAL COMPANY, INC., Chiyoda-ku, Tokyo (JP)
(72) Inventor: Doya, Masaharu, c/o Mitsubishi Gas Chem. Co. Inc., Tayuhama, Niigata-shi, Niigata-ken (JP)
(74) Representative: Kraus, Walter, Dr.

(56) References cited:
- PATENT ABSTRACTS OF JAPAN vol. 97, no. 6, 30 June 1997 & JP 09 048769 A (MITSUBISHI GAS CHEM CO INC), 18 February 1997,
- R. W. STOUGHTON: JOURNAL OF THE AMERICAN CHEMICAL SOCIETY, vol. 63, September 1941, pages 2376-9, XP002076891

## Description

### 1. Field of the invention

The present invention relates to a process for producing a 2,4-oxazolidinedione. More specifically, the present invention relates to a process for producing a purified 2,4-oxazolidinedione using a 2-hydroxycarboxylic acid ester and urea as starting materials. A 2,4-oxazolidinedione is an important substance as, for example, an intermediate for production of a photographic yellow coupler or as an intermediate for producing medications.

### 2. Description of the Prior Art

As a method for producing a purified 2,4-oxazolidinedione using a 2-hydroxycarboxylic acid ester and urea as starting materials, a method is known which comprises heating a 2-hydroxycarboxylic acid ester and urea in ethanol in the presence of an equivalent amount of sodium ethylate to obtain a sodium salt of a 2,4-oxazolidinedione, dissolving this in water, neutralizing the solution with an acid, then extracting the reaction solution with ether, and distilling the extract to obtain a purified 2,4-oxazolidinedione [Journal of American Chemical Society (JACS), vol. 63, pp. 2376 - 2379 (1941)].

In the conventional method for producing a purified 2,4-oxazolidinedione, a costly alkali metal compound has to be used in an equivalent amount. Therefore, a 2,4-oxazolidinedione obtained by the reaction is an alkali metal salt, and neutralization with an acid, extraction with a solvent for desalting, an intricate purification procedure such as distillation or the like is required to obtain a free 2,4-oxazolidinedione.

It is an object of the present invention to provide a process for producing a purified 2,4-oxasolidinedione in a high yield without using a costly alkali metal compound and easily by a simple purification method.

### SUMMARY OF THE INVENTION

The present inventor assiduously conducted investigations on a process for producing a 2,4-oxazolidinedione which involves the above-mentioned problems, and consequently found that a 2,4-oxazolidinedione can easily be obtained in a high yield by previously reacting a 2-hydroxycarboxylic acid ester with urea in the presence of a catalyst at a temperature of from 100 to 250 °C without using a costly alkali metal compound, and filed a patent application (Japanese Patent Laid-Open No. 48,769/1997).

The present inventor has further conducted investigations, and has consequently found that the above-obtained reaction solution contains a 2-hydroxycarboxylic acid amide and carbamic acid ester as by-products, that the 2-hydroxycarboxylic acid amide of these is hardly separated from the 2,4-oxazolidinedione, and that an unreacted 2-hydroxycarboxylic acid ester and the catalyst are separated from the reaction solution through distillation, and the residue is then crystallized or extracted using water to easily obtain a purified 2,4-oxazolidinedione. These findings have led to the completion of the present invention.

That is, the present invention is a process for producing a purified 2,4-oxazolidinedione from a 2-hydroxycarboxylic acid ester and urea, which comprises
[A] a step of reacting a 2-hydroxycarboxylic acid ester represented by formula (1) 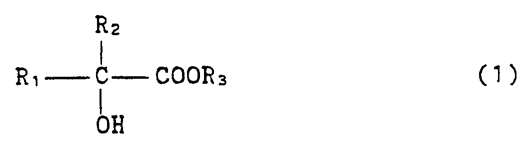 wherein R₁ and R₂, independently from each other, represent a hydrogen atom, a methyl group, an ethyl group, a propyl group, a butyl group, a phenyl group, a benzyl group or a 2-thienyl group, and R₃ represents a lower alkyl group
   with urea in the presence of a catalyst at a temperature of from 100 to 250°C to form a 2,4-oxazolidinedione represented by formula (2) 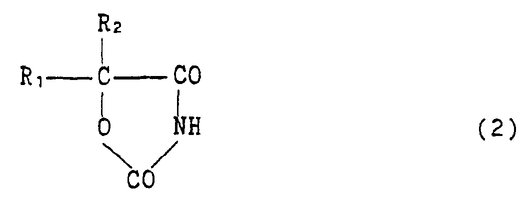 wherein R₁ and R₂ have the same meaning as in formula (1),
   characterized in that the process further comprises
[B] a step of separating the reaction solution obtained in step [A] into (a) a low-boiling fraction composed mainly of an unreacted 2-hydroxycarboxylic acid ester, (b) a fraction composed mainly of the 2,4-oxazolidinedione and (c) a catalyst-containing high-boiling component through distillation, and
[C] a step in which the fraction (b) composed mainly of the 2,4-oxazolidinedione obtained in step [B] is dissolved in water at a weight ratio of water:said fraction (b) of 2 or less and cooled to precipitate crystals of the 2,4-oxazolidinedione, then filtered to separate the 2,4-oxazolidinedione.

### DETAILED DESCRIPTION OF THE INVENTION

The reaction in step [A] of the present invention is conducted by adding a catalyst to a mixed solution of a 2-hydroxycarboxylic acid ester and urea and then heating the mixture.

Examples of the 2-hydroxycarboxylic acid ester used as a starting material include lower alkyl esters of 2-hydroxycarboxylic acids such as glycolic acid, lactic acid, 2-hydroxybutyric acid, 2-hydroxyisobutyric acid, 2-hydroxyvaleric acid, 2-hydroxyhexanoic acid, mandelic acid, 2-hydroxy-3-phenylpropionic acid and 2-(2-thienyl)glycolic acid.

With respect to a ratio of the starting 2-hydroxycarboxylic acid ester to urea, it is advisable that the ratio of the 2-hydroxycarboxylic acid ester is in the range of from 1 to 5 mols per mol of urea. When the molar ratio of the 2-hydroxycarboxylic acid ester to urea is less than 1, the selectivity for the 2,4-oxazolidinedione is decreased by the side reaction of urea itself. Further, when the molar ratio of the 2-hydroxycarboxylic acid ester to urea is more than 5, the amount of the 2-hydroxycarboxylic acid ester to be recovered and recycled is increased, and it is thus economically undesirable.

The reaction in step [A] proceeds upon setting the reaction temperature at 100°C or higher without using the catalyst. However, it is preferable to use the catalyst.

The catalyst is preferably one or more simple metals selected from copper, beryllium, calcium, barium, zinc, cadmium, aluminum, titanium, tin, lead, vanadium, antimony, bismuth, chromium, molybdenum, tellurium, manganese and iron or compounds thereof. In general, a metal powder, a metal plate, an oxide, a hydroxide, an inorganic salt, a carbonate, a basic carbonate and an organic acid salt are used.

These catalysts can be used either singly or in combination. Further, these catalysts can also be used by being mixed with compounds or supports which are inactive to the reaction or by being carried thereon.

The amount of the catalyst is not particularly limited. In general, it is used such that the amount of the metal compound is between 0.0001 and 10 mols, preferably between 0.001 and 1 mol per mol of urea.

The reaction in step [A] is conducted by maintaining a mixture of a 2-hydroxycarboxylic acid ester, urea and a catalyst at a reaction temperature, and simultaneously removing ammonia formed from the reaction mixture.

In order to remove ammonia, a method in which an inert gas is introduced into a reaction solution under reaction conditions during the reaction is also effective. A method in which the reaction is conducted while refluxing the starting material or the solvent instead of the inert gas is more effective. When the starting material or the solvent has a high boiling point, it is advisable to conduct refluxing under reduced pressure.

The amount of the starting material or the solvent to be refluxed is between 1 and 100 mols per mol of ammonia formed.

The reaction temperature is between 100 and 250°C, preferably between 120 and 200°C . When the reaction temperature is too low, a reaction rate is low. When it is too high, the rate of the side reaction is increased.

The reaction time varies depending on the type of the starting 2-hydroxycarboxylic acid ester, the type and the amount of the catalyst, and the reaction temperature. It is usually between 0.5 and 20 hours.

Step [B] of separating the unreacted 2-hydroxycarboxylic acid ester and the catalyst from the reaction solution is an ordinary distillation step. First, a low-boiling fraction composed mainly of an unreacted 2-hydroxycarboxylic acid ester which is a lower-boiling substance than the 2,4-oxazolidinedione, and the fraction composed mainly of the 2,4-oxazolidinedione is then obtained. The low-boiling fraction composed mainly of the unreacted 2-hydroxycarboxylic acid ester and the catalyst-containing high-boiling component (still-bottom product) which are recovered are recycled to the reaction system and reused.

The fraction composed mainly of the 2,4-oxazolidinedione obtained in step [B] contains a 2-hydroxycarboxylic acid amide, a carbamic acid ester and the like which are formed by the reaction as by-products, and these by-products are separated and purified in the next step [C].

With respect to the distillation conditions, either normal pressure or reduced pressure is available, and the pressure varies depending on the starting 2-hydroxycarboxylic acid ester and the 2,4-oxazolidinedione formed.

In step [C] of the present invention, the purified 2,4-oxazolidinedione is obtained from the fraction composed mainly of the 2,4-oxazolidinedione as obtained in step [B] by a crystallization method.

In this method, the solvent is added to the fraction composed mainly of the 2,4-oxazolidinedione obtained in step [B], and dissolved therein to conduct crystallization, thereby obtaining the purified 2,4-oxazolidinedione. The solvent used in this crystallization method is preferably water.

When water is used and the amount thereof is large, a ratio of crystals of the purified 2,4-oxazolidinedione obtained is decreased. Accordingly, the amount of water is 2 or less in terms of the weight ratio based on the fraction composed mainly of the starting 2,4-oxazolidinedione. When the amount of water is too small, the purity of the purified 2,4-oxazolidinedione is decreased.

The dissolution temperature after the addition of water varies depending on the types of the 2,4-oxazolidinedione and the by-product contained.

It is usually at most 100°C which is the boiling point of water under normal pressure.

A method for precipitating crystals of the 2,4-oxazolidinedione may be an ordinary cooling method. At this time, the cooling temperature is preferably as low as possible to obtain a high yield of crystals.

The primary crystals of the 2,4-oxazolidinedione obtained by the filtration after the cooling contain a small amount of a filtrate containing by-products. Accordingly, in order to obtain a high-purity 2,4-oxazolidinedione, it is effective to wash the crystals with a small amount of water. The amount of water used to wash the crystals varies depending on the type of the 2,4-oxazolidinedione.

Water is ordinarily used in an amount of 1 or less in terms of the weight ratio based on the primary crystals of the 2,4-oxazolidinedione. The temperature of water used is preferably as low as possible to reduce the loss of dissolution of the 2,4-oxazolidinedione crystals in water.

Since the thus-obtained crystals of the 2,4-oxazolidinedione contain a small amount of water used in washing, the purified 2,4-oxazolidinedione is obtained by removing water through drying or distillation.

The filtrate separated through crystallization contains the 2,4-oxazolidinedione and a 2-hydroxycarboxylic acid amide, a carbamic acid ester and the like which are by-products. The 2,4-oxazolidinedione is recovered from this filtrate through extraction with the solvent, or water is separated from the filtrate through distillation and the residue is then recycled to the reaction system, or the filtrate is reacted with urea or a carbonic acid diester, making it possible to improve the yield of the purified 2,4-oxazolidinedione.

The wash liquid obtained by washing the primary crystals is used as water to be added first in the crystallization, whereby the total amount of the 2,4-oxazolidinedione dissolved in the wash liquid is recovered, and the yield of the purified 2,4-oxazolidinedione can thus be improved.

Each of steps represented by [A] to [C] in the present invention can be conducted either batchwise or continuously.

In accordance with the process of the present invention, the purified 2,4-oxazolidinedione can be produced from the 2-hydroxycarboxylic acid ester and urea by a simple purification method without using a costly alkali metal compound.

Therefore, the present invention can provide the purified 2,4-oxasolidinedione important as an intermediate for production of a photographic yellow coupler or as an intermediate for production of medications industrially advantageously.

The present invention is illustrated more specifically by referring to the following Examples. However, the present invention is not limited to these Examples.

### DESCRIPTION OF THE PREFERRED EMBODIMENTS

### Example 1

### (step [A])

A 500-milliliter three-necked flask fitted with a stirrer, a reflux condenser and a thermometer was charged with 236.3 g (2.00 mols) of methyl 2-hydroxyisobutyrate, 60.1 g (1.00 mol) of urea and 1.5 g of lead oxide, and the mixture was heat-refluxed at 140°C and 93.3 kPa (700 torr) while being stirred. After the reaction was conducted for 3 hours, the temperature of the reaction solution reached 170 °C. Thereaction solution was cooled to obtain 247.7 g of the reaction solution. The composition of the reaction solution was analyzed through liquid chromatography. Consequently, unreacted methyl 2-hydroxyisobutyrate was 104.3 g, 5,5-dimethyl-2,4-oxazolidinedione formed was 121.9 g, by-product 2-hydroxyisobutyric acid amide was 11.8 g, and by-product methyl carbamate was 3.1 g.

As a result, the conversion of methyl 2-hydroxyisobutyrate was 55.8% relative to the theoretical value of 50.0%, and the yield of 5,5-dimethyl-2,4-oxazolidinedione based on methyl 2-hydroxybutyrate reacted was 84.7%.

### (step [B])

The total amount of the reaction solution obtained in step [A] was charged into a 300-milliliter distillation flask, and the distillation was conducted under reduced pressure to obtain 104.8 g of a low-boiling fraction (< 2.66 kPa (< 20 torr), 50°C) composed mainly of methyl 2-hydroxyisobutyrate, 118.7 g of a fraction (2.66 kPa (20 torr), 50°C ∼ 1.6 kPa (12 torr), 154°C) composed mainly of 5,5-dimethyl-2,4-oxazolidinedione and 20.5 g of a catalyst-containing high-boiling component (still-bottom product).

When the compositions of the each fractions and the still-bottom product were analyzed through liquid chromatography, methyl 2-hydroxyisobutyrate was 103.1 g in the low-boiling fraction; 5,5-dimethyl-2,4-oxazolidinedione was 104.4 g, 2-hydroxyisobutyric acid amide was 9.7 g, methyl carbamate was 1.3 g in the main fraction; and 5,5-dimethyl-2,4-oxazolidinedione was 17.4 g in the still-bottom product.

### (step [C])

The total amount of the fraction composed mainly of 5,5-dimethyl-2,4-oxazolidinedione obtained in step [B] and 70 g of water were charged into a 300-milliliter beaker, heated at 50 °C, and uniformly dissolved. The solution was then cooled to 5 °C. The crystals precipitated were filtered, and the filtrate was separated. The resulting primary crystals were then washed with 50 g of cold water of 5°C , and dried to obtain 85.2 g of purified 5,5-dimethyl-2,4-oxazolidinedione. The filtrate recovered was 70.9 g, and the wash liquid recovered was 72.8 g.

The purity of the resulting purified 5,5-dimethyl-2,4-oxazolidinedione and the compositions of the filtrate and the wash liquid were analyzed through liquid chromatography. Consequently, the purity of purified 5,5-dimethyl-2,4-oxazolidinedione was 99.3%; 5,5-dimethyl-2,4-oxazolidinedione was 11.2 g, 2-hydroxyisobutyric acid amide was 7.7 g, methyl carbamate was 1.0 g in the filtrate; 5,5-dimethyl-2,4-oxazolidinedione was 8.1 g, 2-hydroxyisobutyric acid amide.was 1.7 g, and methyl carbamate was 0.2 g in the wash liquid. The net yield of 5,5-dimethyl-2,4-oxazolidinedione which was provided in consideration of the purity of purified 5,5-dimethyl-2,4-oxazolidinedione based on starting urea was 65.5%.

### Example 2

### (step [A])

The same device as that used in Example 1 was charged with 130.0 g (1.10 mols) of methyl 2-hydroxyisobutyrate, 60.1 g (1.00 mol) of urea, and the total amount of the low-boiling fraction composed mainly of methyl 2-hydroxyisobutyrate and the catalyst-containing high-boiling component (still-bottom product) which were recovered in step [B] of Example 1. The reaction was conducted for 3 hours as in Example 1. The reaction solution was cooled to obtain 269.0 g of the reaction solution. The composition of the reaction solution was analyzed through liquid chromatography. As a result, unreacted methyl 2-hydroxyisobutyrate was 109.0 g, 5,5-dimethyl-2,4-oxazolidinedione formed was 130.5 g, by-product 2-hydroxyisoburyric acid amide was 14.5 g, and by-product methyl carbamate was 5.5 g.

Consequently, the conversion of methyl 2-hydroxyisobutyrate was 53.2% relative to the theoretical value of 50.0%, and the yield of 5,5-dimethyl-2,4-oxazolidinedione formed freshly on the basis of methyl 2-hydroxyisobutyrate reacted was 83.4%.

### (step [B])

The total amount of the reaction solution obtained in step [A] was charged into a 300-milliliter distillation flask, and the distillation was conducted under reduced pressure to obtain 107.2 g of a low-boiling fraction (< 2.66 kPa (< 20 torr), 120°C) composed mainly of methyl 2-hydroxyisobutyrate, 133.6 g of a fraction (2.66 kPa (20 torr), 120°C ∼ 1.6 kPa (12 torr), 154°C) composed mainly of 5,5-dimethyl-2,4-oxazolidinedione and 23.5 g of a catalyst-containing high-boiling component (still-bottom product).

The compositions of the each fractions and the still-bottom product were analyzed through liquid chromatography. Consequently, methyl 2-hydroxyisobutyrate was 105.0 g in the low-boiling fraction; 5,5-dimethyl-2,4-oxazolidinedione was 114.8 g, 2-hydroxyisobutyric acid amide was 12.2 g, methyl carbamate was 3.2 g in the main fraction; and 5,5-dimethyl-2,4-oxazolidinedione was 19.7 g in the still-bottom product.

### (step [C])

A 300-milliliter beaker was charged with the total amount of the fraction composed mainly of 5,5-dimethyl-2,4-oxazolidinedione obtained in step [B] and the total amount of the wash liquid recovered in step [C] of Example 1. The mixture was heated at 50°C, and uniformly dissolved. Then, the solution was cooled to 5°C . The crystals precipitated were filtered to separate the filtrate. The resulting primary crystals were washed with 50 g of cold water of 5°C to obtain 97.3 g of purified 5,5-dimethyl-2,4-oxazolidinedione. The filtrate recovered was 82.4 g, and the wash liquid recovered was 55.7 g.

The purity of the resulting purified 5,5-dimethyl-2,4-oxazolidinedione and the compositions of the filtrate recovered and the wash liquid recovered were analyzed through liquid chromatography. Consequently, the purity of purified 5,5-dimethyl-2,4-oxazolidinedione was 99.2%; 5,5-dimethyl-2,4-oxazolidinedione was 15.7 g, 2-hydroxyisobutyric acid amide was 12.1 g, methyl carbamate was 2.9 g in the filtrate; 5,5-dimethyl-2,4-oxazolidinedione was 9.8 g, 2-hydroxyisobutyric acid amide was 1.8 g, and methyl carbamate was 0.5 g in the wash liquid.

The net yield of 5,5-dimethyl-2,4-oxazolidinedione based on starting urea was 74.8%.

### Example 3

Fifty milliliter of diisopropyl ether were added to the total amount of the filtrate after the crystallization and the separation which was recovered in step [C] of Example 2 to extract 5,5-dimethyl-2,4-oxazolidinedione. The extraction procedure was conducted twice, and the resulting extract was concentrated to recover 14.9 g of 5,5-dimethyl-2,4-oxazolidinedione. When this was combined with purified 5,5-dimethyl-2,4-oxazolidinedione obtained in step [C] of Example 2, the amount was 112.2 g, and the yield thereof based on starting urea was 86.3%.

### Example 4 (Step [C] of this example does not constitute an embodiment of Step [C] as defined in claim 1)

### (step [A])

The same device as that used in Example 1 was charged with 354.4 g (3.00 mols) of ethyl lactate, 60.1 g (1.00 mol) of urea and 3.0 g of zinc oxide, and the mixture was heat-refluxed at 155 °C under normal pressure while being stirred. After the reaction was conducted for 2 hours, the temperature of the reaction solution reached 165 °C. The reaction solution was cooled to obtain 348.6 g of the reaction solution. The composition of the reaction solution was analyzed through liquid chromatography. Consequently, unreacted ethyl lactate was 226.8 g, 5-methyl-2,4-oxazolidinedione formed was 106.5 g, by-product lactic acid amide was 6.7 g, and by-product ethyl carbamate was 2.9 g.

As a result, the conversion of ethyl lactate was 36.0% relative to the theoretical value of 33.3%, and the yield of 5-methyl-2,4-oxazolidinedione based on ethyl lactate was 85.7%.

### (step [B])

A 500-milliliter distillation flask was charged with the total amount of the reaction solution obtained in step [A], and the distillation was conducted under reduced pressure to obtain 228.9 g of a low-boiling fraction (< 2.66 kPa (< 20 torr), 120°C) composed mainly of ethyl lactate, 100.4 g of a fraction (2.66 kPa (20 torr), 120°C ∼ 0.66 kPa (5 torr), 140°C) composed mainly of 5-methyl-2,4-oxazolidinedione and 16.8 g of a catalyst-containing high-boiling component.

The compositions of the each fractions and the still-bottom product were analyzed through liquid chromatography. As a result, ethyl lactate was 225.8 g in the low-boiling fraction; 5-methyl-2,4-oxazolidinedione was 94.3 g, lactic acid amide was 5.7 g in the main fraction; ethyl carbamate was 0.9 g, and 5-methyl-2,4-oxazolidinedione was 12.4 g in the still-bottom product.

### (step [C])

A 500-milliliter separatory funnel was charged with the total amount of the fraction composed mainly of 5-methyl-2,4-oxazolidinedione obtained in step [B], 200 g of methyl-t-butyl ether and 100 g of water. The solution was separated while being shaken. The organic phase of the upper layer obtained was further washed twice with water in an amount of 100 g for each washing. Subsequently, methyl-t-butyl ether was removed through distillation to obtain 64.2 g of purified 5-methyl-2,4-oxazolidinedione.

Further, the aqueous phase of the lower layer was combined therewith, and extracted three times with methyl-t-butyl ether in an amount of 70 g for each extraction to recover 5-methyl-2,4-oxazolidinedione in the aqueous phase.

The resulting purified 5-methyl-2,4-oxazolidinedione and the composition of the methyl-t-butyl ether solution containing 5-methyl-2,4-oxazolidinedione which was recovered through extraction of the aqueous phase were analyzed through liquid chromatography. As a result, the purity of purified 5-methyl-2,4-oxazolidinedione was 99.6%; 5-methyl-2,4-oxazolidinedione was 28.7 g, lactic acid amide was 0.5 g, and ethyl carbamate was 0.2 g in the recovered methyl-t-butyl ether solution containing 5-methyl-2,4-oxazolidinedione.

The net yield of 5-methyl-2,4-oxazolidinedione based on starting urea was 55.6%.

### Example 5 (Step [C] of this example does not constitute an embodiment of Step [C] as defined in claim 1)

### (step [A])

The same device as that used in Example 1 was charged with 128.6 g (1.09 mols) of ethyl lactate, 60.1 g (1.00 mol) of urea, and the total amount of the low-boiling fraction composed mainly of ethyl lactate and the catalyst-containing high-boiling still-bottom product which were recovered in step [B] of Example 4. The reaction was conducted for 2 hours as in Example 1. The reaction solution was cooled to obtain 368.8 g of the reaction solution. The composition of the reaction solution was analyzed through liquid chromatography. Consequently, unreacted ethyl lactate was 228.9 g, 5-methyl-2,4-oxazolidinedione formed was 115.9 g, by-product lactic acid amide was 7.8 g, and by-product ethyl carbamate was 3.3 g.

As a result, the conversion of ethyl lactate was 35.4% relative to the theoretical value of 33.3%. The yield of 5-methyl-2,4-oxazolidinedione formed freshly on the basis of ethyl lactate reacted was 84.7%.

### (step [B])

A 500-milliliter distillation flask was charged with the total amount of the reaction solution obtained in step [A], and the distillation was conducted under reduced pressure to obtain 229.3 g of a low-boiling fraction (< 2.66 kPa (< 20 torr), 120°C) composed mainly of ethyl lactate, 118.7 g of a fraction (2.66 kRa (20 torr), 120°C ∼ 0.66 kPa (5 torr), 140°C) composed mainly of 5-methyl-2,4-oxazolidinedione and 18.8 g of a catalyst-containing high-boiling fraction (still-bottom product).

The compositions of the each fractions and the still-bottom product were analyzed through liquid chromatography. As a result, ethyl lactate was 226.2 g in the low-boiling fraction; 5-methyl-2,4-oxazolidinedione was 106.4 g, lactic acid amide was 6.6 g, ethyl carbamate was 1.2 g in the main fraction; and 5-methyl-2,4-oxazolidinedione was 10.1 g in the still-bottom product.

### (step [C])

The total amount of the methyl-t-butyl ether solution containing 5-methyl-2,4-oxazolidinedione which was recovered in step [C] of Example 4 and 100 g of water were added to the total amount of the fraction composed mainly of 5-methyl-2,4-oxazolidinedione obtained in step [B] in a 500-milliliter separatory funnel, and the solution was separated while being shaken. The organic layer of the resulting upper layer was further washed twice with water in an amount of 100 g for each washing. Subsequently, methyl-t-butyl ether was removed through distillation to obtain 93.7 g of purified 5-methyl-2,4-oxazolidinedione.

Further, the aqueous phase of the lower layer was combined therewith, and extracted twice with methyl-t-butyl ether in an amount of 100 g for each extraction.

The resulting purified 5-methyl-2,4-oxazolidinedione and the composition of the methyl-t-butyl ether solution containing 5-methyl-2,4-oxazolidinedione recovered through extraction of the aqueous phase were analyzed through liquid chromatography. Consequently, the purity of purified 5-methyl-2,4-oxazolidinedione was 99.4%; 5-methyl-2,4-oxazolidinedione was 39.5 g, lactic acid amide was 0.7 g, and ethyl carbamate was 0.3 gin the recovered methyl-t-butyl ether solution containing 5-methyl-2,4-oxazolidinedione.

The net yield of 5-methyl-2,4-oxazolidinedione based on starting urea was 80.9%.

## Claims

1. A process for producing a purified 2,4-oxazolidinedione from a 2-hydroxycarboxylic acid ester and urea, which comprises
[A] a step of reacting a 2-hydroxycarboxylic acid ester represented by formula (1) 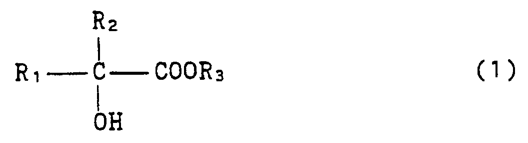 wherein R₁ and R₂, independently from each other, represent a hydrogen atom, a methyl group, an ethyl group, a propyl group, a butyl group, a phenyl group, a benzyl group or a 2-thienyl group, and R₃ represents a lower alkyl group
with urea in the presence of a catalyst at a temperature of from 100 to 250°C to form a 2,4-oxazolidinedione represented by formula (2) 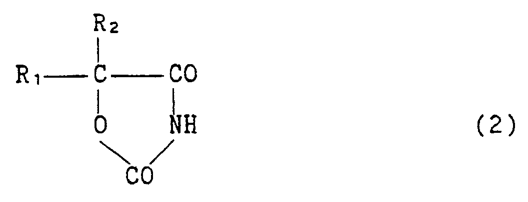 wherein R₁ and R₂ have the same meaning as in formula (1),
**characterized in that** the process further comprises
[B] a step of separating the reaction solution obtained in step [A] into (a) a low-boiling fraction composed mainly of an unreacted 2-hydroxycarboxylic acid ester, (b) a fraction composed mainly of the 2,4-oxazolidinedione and (c) a catalyst-containing high-boiling component through distillation, and
[C] a step in which the fraction (b) composed mainly of the 2,4-oxazolidinedione obtained in step [B] is dissolved in water at a weight ratio of water:said fraction (b) of 2 or less and cooled to precipitate crystals of the 2,4-oxazolidinedione, then filtered to separate the 2,4-oxazolidinedione.

2. The process for producing the 2,4-oxazolidinedione according to claim 1, wherein the catalyst used in step [A] is one or more simple metals selected from copper, beryllium, calcium, barium, zinc, cadmium, aluminum, titanium, tin, lead, vanadium, antimony, bismuth, chromium, molybdenum, tellurium, manganese and iron or compounds thereof.

3. The process for producing the 2,4-oxazolidinedione according to claim 1, wherein (a) the low-boiling fraction composed mainly of the unreacted 2-hydroxycarboxylic acid ester and (c) the catalyst-containing high-boiling component separated in step [B] are recycled and reused.

4. The process for producing the 2,4-oxazolidinedione according to claim 1, wherein in step [C] the filtrate is extracted with an organic solvent to obtain the lesser part of the 2,4-oxazolidinedione.

5. The process for producing the 2,4-oxazolidinedione according to claim 1, wherein in step [C] the 2,4-oxazolidinedione is rinsed with water, and the rinsed water is used as dissolving water.

## Patentansprüche

1. Verfahren zur Herstellung eines gereinigten 2,4-Oxazolidindions aus einem 2-Hydroxycarbonsäureester und Harnstoff, umfassend:
[A] eine Stufe der Umsetzung eines 2-Hydroxycarbonsäureesters, dargestellt durch die Formel (1) 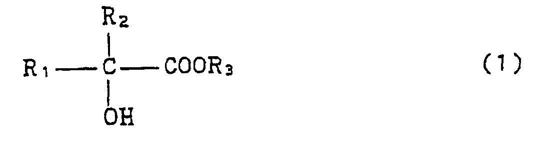 worin R₁ und R₂, unabhängig voneinander, ein Wasserstoffatom, eine Methylgruppe, eine Ethylgruppe, eine Propylgruppe, eine Butylgruppe, eine Phenylgruppe, eine Benzylgruppe oder eine 2-Thienylgruppe darstellen und R₃ eine Niedrigalkylgruppe darstellt,
mit Harnstoff in Gegenwart eines Katalysators bei einer Temperatur von 100 bis 250°C unter Bildung eines 2,4-Oxazolidindions, das durch die Formel (2) dargestellt wird 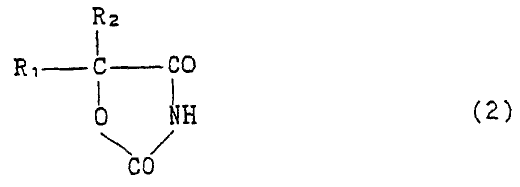 worin R₁ und R₂ dieselbe Bedeutung wie in Formel (1) haben,
**dadurch gekennzeichnet,**
**dass** das Verfahren außerdem umfasst:
[B] eine Stufe der Trennung der in Stufe [A] erhaltenen Reaktionslösung in (a) eine niedrigsiedende Fraktion, die hauptsächlich aus einem nicht umgesetzten 2-Hydroxycarbonsäureester besteht, (b) eine Fraktion, die hauptsächlich aus dem 2,4-Oxazolidindion besteht, und (c) eine Katalysator-enthaltende hochsiedende Komponente durch die Destillation und
[C] eine Stufe, in der die Fraktion (b), die hauptsächlich aus dem in Stufe [B] erhaltenen 2,4-Oxazolidindion besteht, in Wasser bei einem Gewichtsverhältnis von Wasser zu dieser Fraktion (b) von 2 oder weniger gelöst wird und gekühlt wird, um Kristalle des 2,4-Oxazolidindions auszufällen, dann filtriert wird, um das 2,4-Oxazolidindion abzutrennen.

2. Verfahren zur Herstellung des 2,4-Oxazolidindions nach Anspruch 1, wobei der in Stufe [A] verwendete Katalysator ein einfaches Metall oder mehrere einfache Metalle, ausgewählt aus Kupfer, Beryllium, Calcium, Barium, Zink, Cadmium, Aluminium, Titan, Zinn, Blei, Vanadium, Antimon, Wismut, Chrom, Molybdän, Tellur, Mangan und Eisen, oder Verbindungen davon, ist.

3. Verfahren zur Herstellung des 2,4-Oxazolidindions nach Anspruch 1, wobei (a) die niedrigsiedende Fraktion, die hauptsächlich aus dem nicht umgesetzten 2-Hydroxycarbonsäureester besteht, und (c) die Katalysator-enthaltende hochsiedende Komponente, die in Stufe [B] getrennt werden, recycelt und wieder verwendet werden.

4. Verfahren zur Herstellung des 2,4-Oxazolidindions nach Anspruch 1, wobei in Stufe [C] das Filtrat mit einem organischen Lösungsmittel unter Erhalt des geringeren Teils des 2,4-Oxazolidindions extrahiert wird.

5. Verfahren zur Herstellung des 2,4-Oxazolidindions nach Anspruch 1, wobei in Stufe [C] das 2,4-Oxazolidindion mit Wasser gespült wird und das Spülwasser als Lösungswasser verwendet wird.

## Revendications

1. Procédé pour la préparatian d'une 2,4-oxazolidinedione purifiée à partir d'un ester d'acide 2-hydroxycarboxylique et de l'urée, qui comprend
[A] une étape de réaction d'un ester d'acide 2-hydroxycarboxylique représenté par la formule (1) 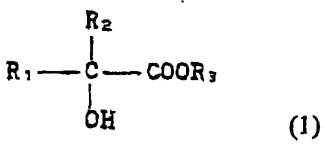 dans laquelle R₁ et R₂, indépendamment l'un de l'autre, représentent un atome d'hydrogène, un groupe méthyle, un groupe éthyle, un groupe propyle, un groupe butyle, un groupe phényle, un groupe benzyle ou un groupe 2-thiényle et R₃ représente un groupe alkyle inférieur
avec l'urée en présence d'un catalyseur à une température allant de 100 à 250° C pour former 2,4-oxazolidinedione représenté par la formule (2) 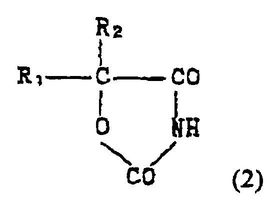 dans laquelle R₁ et R₂ ont la même signification que dans la formule (1),
**caractérisé en ce que** le procédé comprend en outre
[B] une étape de séparation de la solution réactionnelle obtenue à l'étape [A] en (a) une fraction à bas point d'ébullition composée principalement d'un ester d'acide 2-hydroxycarboxylique n'ayant pas réagi, (b) une fractions composée principalement de la 2,4-oxazolidinedione et (c) un composant contenant un catalyseur à point d'ébullition élevé par l'intermédiaire de la distillation, et
[C] une étape dans laquelle la fraction (b) composée principalement de la 2,4-oxazolidinedione obtenue à l'étape [B] est dissoute dans l'eau selon un rapport en poids d'eau / ladite fraction (b) de 2 ou moins et refroidie pour précipiter des cristaux de la 2,4-oxazolidinedione, puis filtrée pour séparer la 2,4-oxazolidinedione.

2. Procédé pour la préparation de 2,4-oxazolidinedione selon la revendication 1, dans lequel le catalyseur utilisé à l'étape [A] est un métal ou plusieurs métaux simples choisis parmi le cuivre, le béryllium, le calcium, le baryum, le zinc, le cadmium, l'aluminium, le titane, l'étain, le plomb, le vanadium, l'antimoine, le bismuth, le chrome, le molybdène, le tellure, le manganèse et le fer, ou des composés de ceux-ci.

3. Procédé pour la préparation de 2,4-oxazolidinedione selon la revendication 1, dans lequel (a) la fraction à bas point d'ébullition composée principalement de l'ester d'acide 2-hydroxycarboxylique n'ayant pas réagi et (c) le composant à point d'ébullition élevé contenant le catalyseur séparé à l'étape [B] sont recyclés et réutilisés.

4. Procédé pour la préparation de 2,4-oxazolidinedione selon la revendication 1, dans lequel, à l'étape [C] le filtrat est extrait avec un solvant organique pour obtenir la partie moindre de 2,4-oxazolidinedione.

5. Procédé pour la préparation de 2,4-oxazolidinedione selon la revendication 1, dans lequel, à l'étape [C] la 2,4-oxazolidinedione est rincée avec de l'eau et l'eau de rinçage est utilisée comme eau dissolvante.
